# EUROPEAN PATENT APPLICATION

(11) **EP 4 494 667 A1**
(43) Date of publication of application: **22.01.2025**
(21) Application number: 23770458.0
(22) Date of filing: 03.03.2023
(51) Int. Cl.: A61L 27/24, A61L 15/32, A61L 15/42, A61L 15/64, A61L 27/56, A61L 27/58

(54) **BIOCOMPATIBLE MEMBER AND METHOD FOR PRODUCING SAME**

(30) Priority: 16.03.2022 JP 2022040999
(71) Applicant: National University Corporation Tokyo Medical and Dental University, Tokyo 113-8510 (JP); Kabushiki Kaisha Toshiba, Tokyo 105-0023 (JP)
(72) Inventor: KIMURA, Tsuyoshi, Tokyo 113-8510 (JP); TOKUNO, Yoko, Tokyo 105-0023 (JP); KISHIDA, Akio, Tokyo 113-8510 (JP); UCHIDA, Kenya, Tokyo 105-0023 (JP)
(74) Representative: Hoffmann Eitle
(86) International application number: PCT/JP2023/007998
(87) International publication number: WO 2023/176501

(57) **Abstract**

A biocompatible member according to an embodiment is a biocompatible member including multiple fibers including a biocompatible material. Mutually adjacent fibers among the fibers are covalently bonded at multiple locations in an extension direction of the fibers.

## Description

### [Technical Field]

Embodiments of the invention relate to a biocompatible member and a method for manufacturing a biocompatible member.

### [Background Art]

For example, biocompatible members for use in the medical field have been proposed. For example, biocompatible members are used *in vivo* or as cell culture scaffoldings. Generally, a biocompatible member has high biocompatibility and is gradually consumed by *in vivo* reactions. By being consumed by *in vivo* reactions, the biocompatible member can be prevented from inhibiting cell growth and/or tissue regeneration.

However, according to the application of the biocompatible member, there are cases where it is favorable for the biocompatible member in an *in vivo* environment (e.g., 37 °C) to remain for a certain period and to have little loss of mechanical strength such as the compressive elastic modulus or the like.

It is therefore desirable to develop technology that can improve longevity and maintain mechanical strength in an *in vivo* environment.

### [Prior Art Documents]

### [Patent Literature]

[Patent Literature 1]
JP-A 2021-122557 (Kokai)

### [Summary of Invention]

### [Problem to be Solved by the Invention]

A problem to be solved by the invention is to provide a biocompatible member and a method for manufacturing a biocompatible member that can improve longevity and maintain mechanical strength in an *in vivo* environment.

### [Means for Solving the Problem]

A biocompatible member according to an embodiment is a biocompatible member that includes multiple fibers including a biocompatible material. Mutually adjacent fibers among the fibers are covalently bonded at multiple locations in an extension direction of the fibers.

### [Brief Description of Drawings]

[FIG. 1]

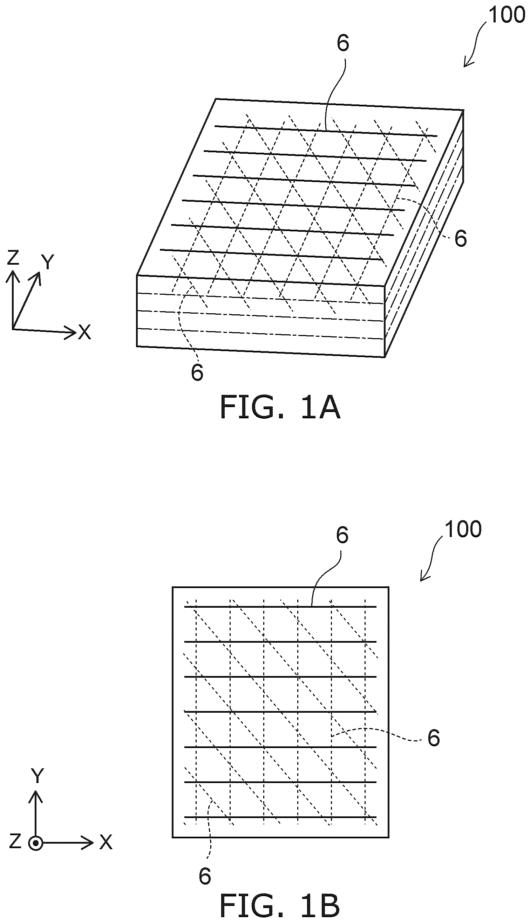

   FIGS. 1A and 1B are schematic views illustrating a biocompatible member.
[FIG. 2]

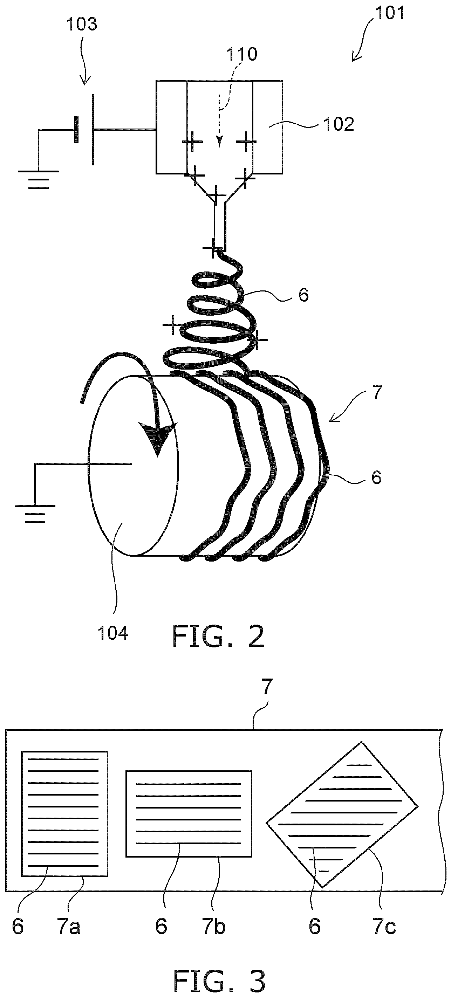

   FIG. 2 is a schematic view illustrating an electrospinning apparatus.
[FIG. 3]
   FIG. 3 is a schematic view illustrating cutouts of a deposited body.
[FIG. 4]

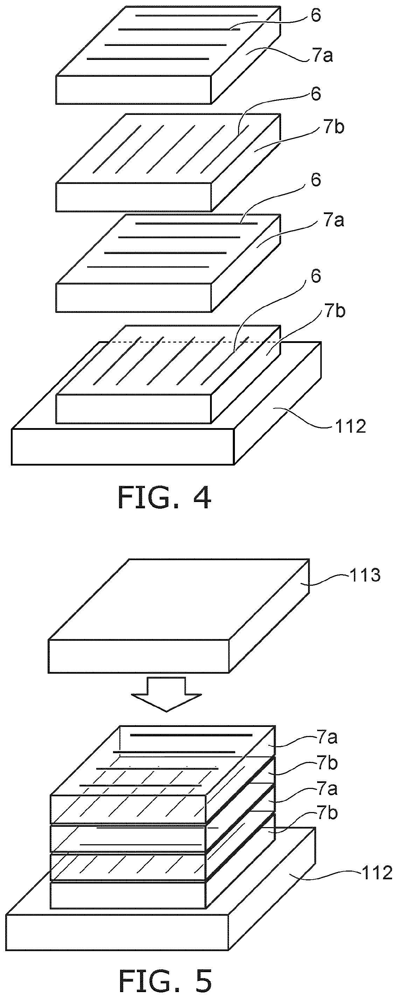

   FIG. 4 is a schematic view illustrating a process of manufacturing a biocompatible member using a deposited body.
[FIG. 5]
   FIG. 5 is a schematic view illustrating a process of manufacturing the biocompatible member using the deposited body.
[FIG. 6]

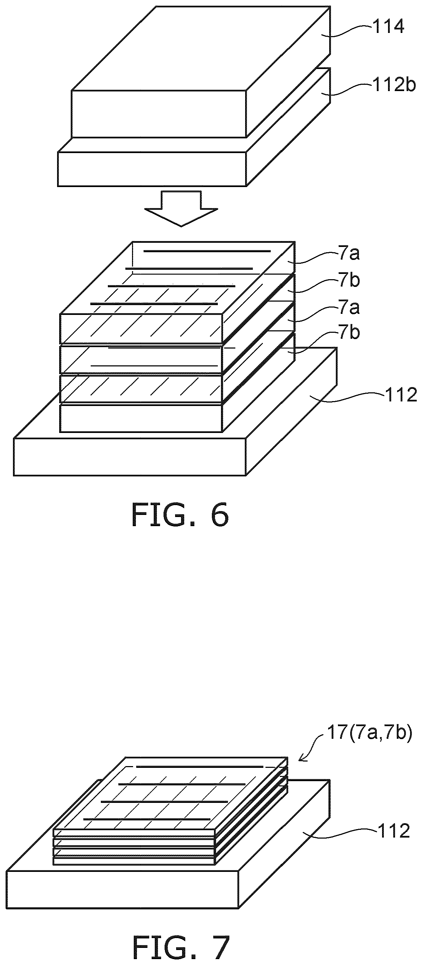

   FIG. 6 is a schematic view illustrating a process of manufacturing the biocompatible member using the deposited body.
[FIG. 7]
   FIG. 7 is a schematic view illustrating a process of manufacturing the biocompatible member using the deposited body.
[FIG. 8]

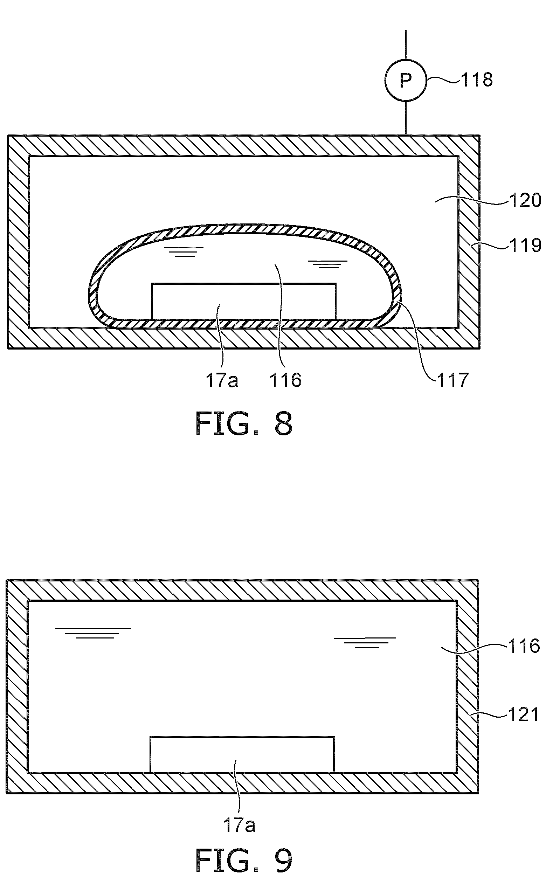

   FIG. 8 is a schematic view illustrating hydrostatic pressure treatment.
[FIG. 9]
   FIG. 9 is a schematic view illustrating decompression treatment.
[FIG. 10]

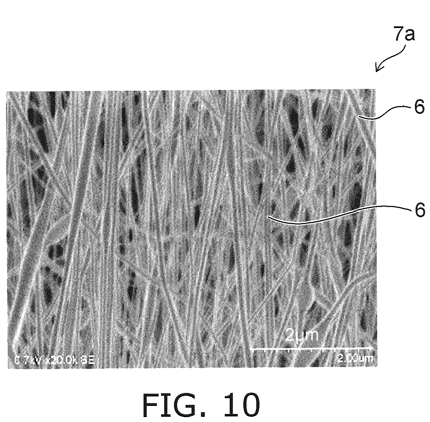

   FIG. 10 is a SEM photograph of a surface of a deposited body.
[FIG. 11]

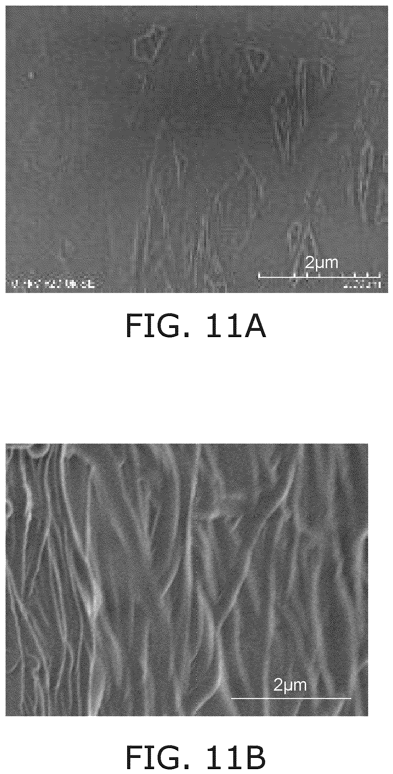

   FIGS. 11A and 11B are SEM photographs of surfaces of aggregates.
[FIG. 12]

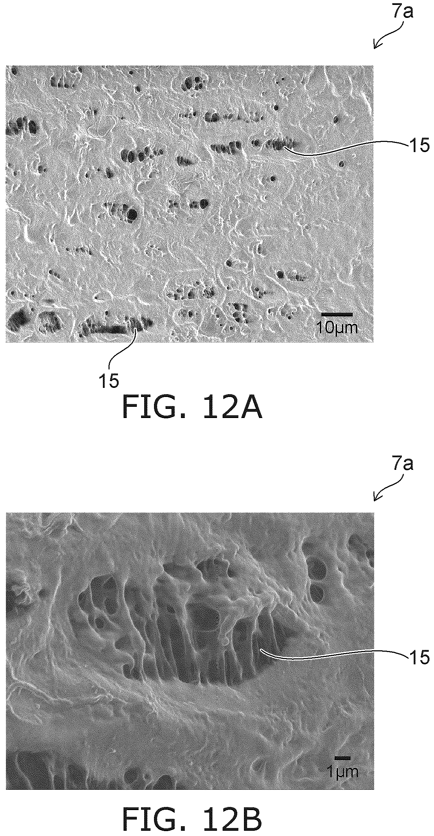

   FIGS. 12A and 12B are SEM photographs of a surface of an aggregate on which hydrostatic pressure treatment and freeze-drying treatment are performed.
[FIG. 13]

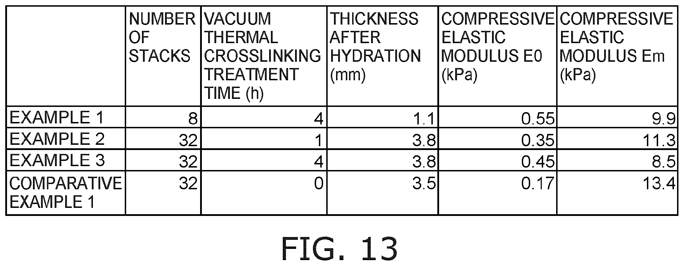

   FIG. 13 is a table showing measurement results of compressive elastic moduli of a biocompatible member 100 according to test examples.
[FIG. 14]

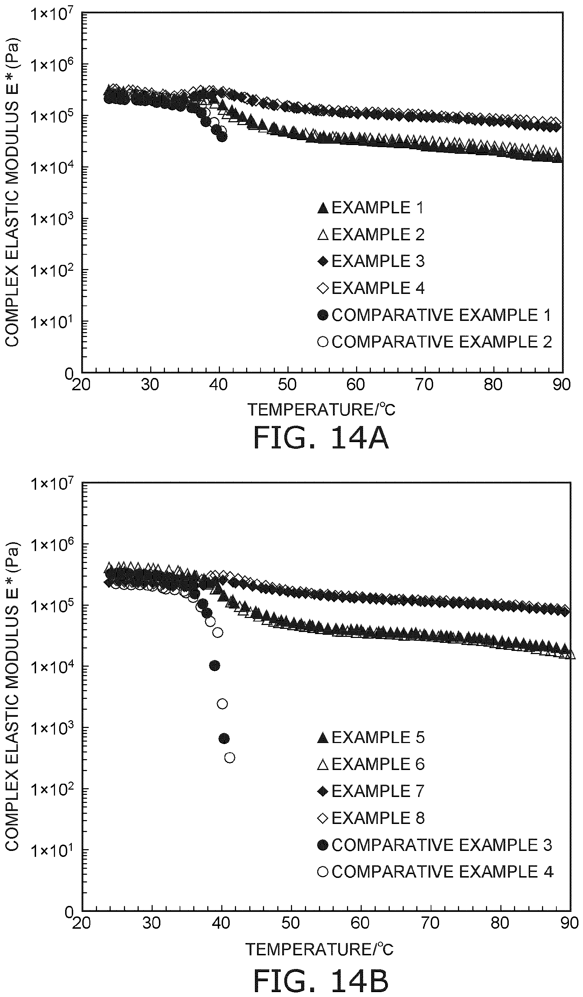

   FIG. 14A is a graph showing measurement results performed in distilled water. FIG. 14B is a graph showing measurement results performed in a physiologic saline solution.
[FIG. 15]

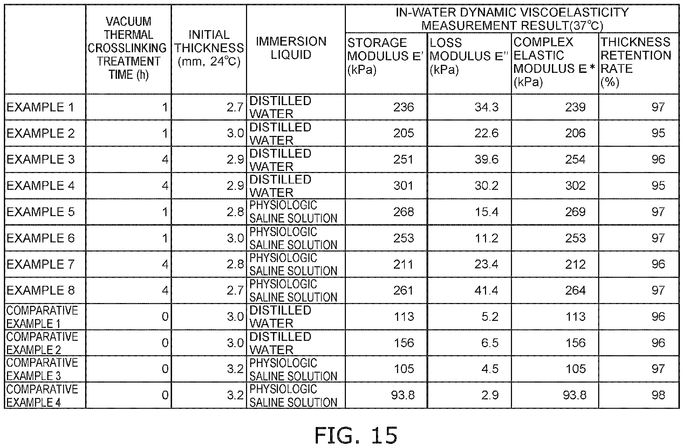

   FIG. 15 is a table summarizing results of dynamic viscoelasticity measurements in water at a temperature of 37 °C.
[FIG. 16]

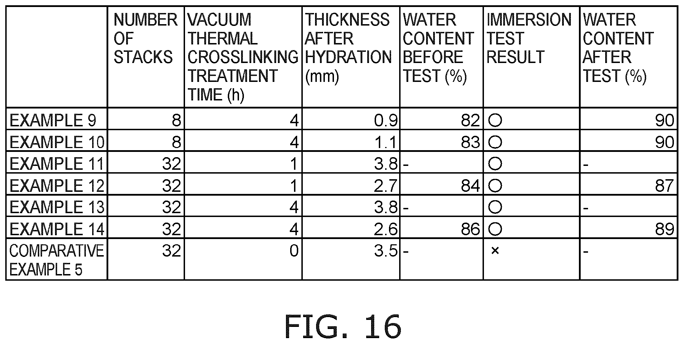

   FIG. 16 is a table showing results of an immersion test of aggregates.

### [Detailed Description]

Embodiments will now be illustrated with reference to the drawings. Similar components in the drawings are marked with the same reference numerals; and a detailed description is omitted as appropriate.

### (Biocompatible member)

FIGS. 1A and 1B are schematic views illustrating the biocompatible member 100.

FIG. 1A is a schematic perspective view of the biocompatible member 100; and FIG. 1B is a drawing of the biocompatible member 100 of FIG. 1A when viewed along the Z-direction.

FIGS. 1A and 1B are schematic views conceptually representing the configuration of the biocompatible member 100, and do not necessarily match the actual configuration. For example, although the diameter of a fiber 6 is extremely small compared to the thickness of the biocompatible member 100 as described below, the fibers 6 are drawn larger than the actual fibers 6 for easier viewing of the drawing. This is similar for the other drawings described below as well.

Also, arrows X, Y, and Z in the drawings represent three mutually-orthogonal directions. For example, the thickness direction of the biocompatible member 100 (a direction perpendicular to a major surface of the biocompatible member 100) is taken as a Z-direction. Also, one direction perpendicular to the thickness direction is taken as a Y-direction; and a direction perpendicular to the Z-direction and Y-direction is taken as an X-direction.

As shown in FIGS. 1A and 1B, the biocompatible member 100 includes the multiple fibers 6. The diameter of the fiber 6 is, for example, about 60 nm to 3 µm. As described below, for example, the fibers 6 can be formed using electrospinning.

In the biocompatible member 100, the extension directions of the fibers 6 are substantially uniform in a prescribed region in the Z-direction (the thickness direction). In other words, in the biocompatible member 100, the fibers 6 extend in about the same direction in a prescribed region in the Z-direction.

Also, in the biocompatible member 100, a first region in which the fibers 6 extend in a first direction and a second region in which the fibers 6 extend in a second direction crossing the first direction are stacked.

For example, in the case of the biocompatible member 100 illustrated in FIGS. 1A and 1B, the fibers 6 extend in the X-direction in the surface region of the biocompatible member 100. Also, below the surface region, a region in which the fibers 6 extend in the Y-direction and a region in which the fibers 6 extend in a direction crossing the X-direction and the Y-direction are provided.

Although FIGS. 1A and 1B illustrate a case where four regions having different extension directions of the fibers 6 are arranged to overlap in the Z-direction, the extension directions of the fibers 6 and the number of stacks of regions having different extension directions of the fibers 6 can be modified as appropriate according to the application of the biocompatible member 100, etc.

For example, regions in which the fibers 6 extend in the X-direction and regions in which the fibers 6 extend in the Y-direction may be stacked alternately in the Z-direction (see, e.g., FIG. 3).

Also, regions having the same extension direction of the fibers 6 may be stacked in the Z-direction.

For example, the extension directions of the fibers 6 and the number of stacks of regions having different extension directions of the fibers 6 can be modified to match the structure of the part of the living body in which the biocompatible member 100 is used. Thus, the biocompatibility of the biocompatible member 100 can be further increased.

For example, when the biocompatible member 100 is used for a tendon or the like of a living body, one region in which the extension directions of the fibers 6 are uniform can be provided. Thus, the biocompatibility of the biocompatible member 100 for the tendon or the like of the living body can be improved.

For example, when the biocompatible member 100 is used for skin or the like of a living body, multiple regions having different extension directions of the fibers 6 can be arranged in the Z-direction. Thus, the biocompatibility of the biocompatible member 100 for the skin or the like of the living body can be improved.

Although FIGS. 1A and 1B illustrate the fibers 6 as being separated from each other in the prescribed regions in the Z-direction to illustrate the extension directions of the fibers 6 being substantially uniform, the adjacent fibers 6 are substantially closely adhered to each other.

Also, the atoms in the biocompatible material (the linear polymer) included in the fibers 6 are directly covalently bonded to each other between the adjacent fibers 6. As described below, the biocompatible member 100 does not include a chemically crosslinked component; and the adjacent fibers 6 of the biocompatible member 100 are thermally crosslinked. Also, for example, in the extension direction of the fibers 6, the adjacent fibers 6 are covalently bonded to each other at multiple locations. Therefore, the adjacent fibers 6 are bonded to each other in a linear shape. There are adjacent fibers 6 not only in the X-direction or Y-direction, but also in the Z-direction.

Also, the covalent bonds between the adjacent fibers 6 occur not only inside each region having the same extension direction of the fibers 6, but also at the boundary between the stacked regions. For example, covalent bonds also occur between the fibers 6 adjacent to each other in the stacking direction (the Z-direction) at the boundary between the stacked regions.

In such a case, the bonding force increases as the covalently bonded locations increase. The extension directions of the fibers 6 are substantially uniform inside one region. Therefore, inside one region, there are many covalently bonded locations between one fiber 6 and one fiber 6 adjacent to the one fiber 6.

In contrast, at the boundary between the stacked regions, the extension direction of the fibers 6 included in one region and the extension direction of the fibers 6 included in another region cross. Therefore, at the boundary between the stacked regions, there are fewer covalently bonded locations between one fiber 6 and one fiber 6 adjacent to the one fiber 6 in the stacking direction. However, at the boundary between the stacked regions, multiple fibers 6 are adjacent to the one fiber 6 in the stacking direction. Therefore, at the boundary between the stacked regions, multiple fibers 6 that are adjacent to one fiber 6 in the stacking direction are covalently bonded to the one fiber 6. As a result, weakening of the bonding force at the boundary between the stacked regions can be suppressed.

The covalent bonding between the adjacent fibers 6 can be performed by crosslinking. In such a case, if the adjacent fibers 6 are covalently bonded by chemical crosslinking, there is a risk that the biocompatible material may be modified or become toxic, causing the biocompatibility to degrade. Also, if the adjacent fibers 6 are covalently bonded to each other simply by thermal crosslinking (also called thermal dehydration crosslinking, heat dehydration crosslinking, etc.), there is a risk that the surface of the fiber 6 may be modified (e.g., oxidized), causing the biocompatibility to degrade.

The biocompatible member 100 according to the embodiment does not include a chemically crosslinked component. In other words, the adjacent fibers 6 are not covalently bonded to each other by chemical crosslinking. Also, the adjacent fibers described above are thermally crosslinked. However, in the biocompatible member 100 according to the embodiment, modification of the surface of the fiber 6 when performing the thermal crosslinking is suppressed. Therefore, the bonding force between the adjacent fibers 6 can be increased, and high biocompatibility can be obtained. Details of the thermal crosslinking are described below.

Also, in the biocompatible member 100, the fibers 6 are substantially closely adhered to each other, and so each fiber 6 cannot always be identified when the biocompatible member 100 is observed using a microscope. Also, the boundaries between the stacked regions cannot always be clearly observed.

Also, voids having diameters of not less than 10 µm may be provided in the surface of the biocompatible member 100, etc., at a density of not less than 1/mm².

For example, by providing voids having diameters of not less than 10 µm in the surface of the biocompatible member 100 at a density of not less than 1/mm², it is easier for cells of the living body to penetrate the voids when the biocompatible member 100 is disposed inside the living body. Then, the cells that penetrate the voids are used as scaffolding, and the cells penetrate further into the biocompatible member 100. Therefore, the biocompatibility of the biocompatible member 100 can be further increased.

Furthermore, the biocompatible member 100 can include a liquid having water as a major component. The liquid having water as a major component is, for example, purified water, a physiologic saline solution, serum such as fetal bovine serum (FBS) or the like, various liquid culture media, protein, RNA, DNA, an aqueous solution including a substrate, a suspension of a cell or the like, a body fluid, or blood. For example, the liquid having water as a major component is stored between the fibers 6. By the biocompatible member 100 including the liquid having water as a major component, the biocompatible member 100 can approach the configuration of the biological tissue at which the biocompatible member 100 is disposed.

For example, the content ratio of the liquid having water as a major component can be set to be not less than 40 mass% and not more than 90 mass%. Thus, the content ratio of the liquid in the biocompatible member 100 can approach the content ratio of the liquid in the biological tissue, and so the biocompatibility of the biocompatible member 100 can be further increased.

Also, if the biocompatible member 100 includes the liquid having water as a major component, the biocompatible member 100 can be disposed *in vivo* and used as a cell culture scaffolding as-is without hydration treatment, etc.

Also, if the biocompatible member 100 includes the liquid having water as a major component, the flexibility of the biocompatible member 100 is increased. It is therefore easy to cut the biocompatible member 100 with scissors and/or a scalpel. Also, when disposing the biocompatible member 100 *in vivo,* damage of the surrounding cellular tissue can be suppressed.

The fiber 6 includes a biocompatible material. The biocompatible material is, for example, a biologically derived material. Biologically derived materials are, for example, materials produced by life activities or materials obtained by processing such materials. Also, the biocompatible material may not be a biologically derived material. For example, the biocompatible material may be a synthetic polymer, a functional protein or synthetic polypeptide obtained by artificial synthesis, etc.

For example, the biocompatible material is a protein such as collagen, laminin, gelatin, or the like, a nucleic acid such as deoxyribonucleic acid (DNA), ribonucleic acid (RNA), or the like, polysaccharides such as chitosan, chitin, hyaluronic acid, alginate, heparin, etc. Also, synthetic polymers used as the biocompatible material are, for example, polyhydroxy acids such as polyethylene (PE), polypropylene (PP), polymethyl methacrylate (PMMA), poly(methacrylic acid 2-hydroxyethyl)(PHEMA), poly(N-isopropylacrylamide) (PNIPAAm), polyvinyl alcohol (PVA), polycyanoacrylate, polyethylene terephthalate (PET), nylon 66, polyurethane (PU), polyethylene glycol (PEG), polyethylene oxide (PEO), polylactic acid (PLA), polyglycolic acid, polycaprolactone, or the like, silicones such as polydimethylsiloxane (PDMS), polydimethylsiloxane (PDMS), etc.

As described above, the biocompatibility of the biocompatible member 100 can be improved by the fiber 6 including a biocompatible material. If the biocompatibility can be improved, for example, the biocompatible member 100 will not be toxic when disposed *in vivo.* Also, a rejection reaction such as inflammation of the living body, etc., can be suppressed.

In such a case, if the biocompatible member 100 is formed of the fibers 6 including higher-order collagen, the rejection reaction can be suppressed more effectively.

Therefore, a case will be described below as an example in which the fibers 6 include higher-order collagen, i.e., collagen having a three-dimensional structure. The structure of a general collagen molecule is a triple helix structure; and the higher-order collagen included in the fibers 6 also has a triple helix structure.

The form of the biocompatible member 100 is, for example, sheet-like. The planar dimensions and the planar shape of the biocompatible member 100 can be modified as appropriate according to the application of the biocompatible member 100, etc.

The thickness of one region in which the extension directions of the fibers 6 are uniform is, for example, about 5 µm to 500 µm. The thickness of the biocompatible member 100 can be modified as appropriate according to the number of stacks. As described above, the number of stacks can be modified as appropriate according to the application of the biocompatible member 100, etc. Generally, the thickness of the biocompatible member 100 is, for example, about 0.03 mm to 50 mm.

The biocompatible member 100 is a solid and is not fluidic. Also, the biocompatible member 100 that includes the liquid having water as a major component is a soft elastic body. Therefore, the biocompatible member 100 can be easily cut with scissors and/or a scalpel. For example, the biocompatible member 100 can be patterned at a medical site by pinching with tweezers, cutting with scissors and/or a scalpel, and bending to match the shape of a wound. In other words, the biocompatible member 100 according to the embodiment is easy to handle.

Also, when the fiber 6 includes higher-order collagen, the biocompatible member 100 is, for example, transparent or semi-transparent. If the biocompatible member 100 is transparent or semi-transparent, it is easy to dispose the biocompatible member 100 to match a damaged tissue part and/or a space after tumor excision.

As described above, the extension directions of the fibers 6 are substantially uniform in a prescribed region in the Z-direction. Also, the adjacent fibers 6 are substantially closely adhered to each other; and the adjacent fibers 6 are covalently bonded to each other. Therefore, by having a dense structure in which the fibers 6 are disposed with high density in the prescribed region, the compressive elastic modulus as a sheet material can be increased.

For example, when the biocompatible member 100 includes a region in which the fibers 6 extend in the two directions of the X-direction and Y-direction, a compressive elastic modulus Em of the biocompatible member 100 in the Z-direction can be not less than 5 kPa.

Also, when measuring a viscoelasticity-temperature curve by a dynamic viscoelastic technique, the storage modulus E' is greater than the loss modulus E" in the region from room temperature (e.g., 23 °C) to 90 °C.

If the compressive elastic modulus of the biocompatible member 100 is low, for example, when the biocompatible member 100 includes a liquid having water as a major component, there are cases where the biocompatible member 100 is weakened and can no longer maintain its shape. For example, when the liquid content ratio is set to a value near that of the biological tissue, the biocompatible member 100 becomes gel-like, etc., and it is difficult to handle the biocompatible member 100.

In such a case, if the biocompatible member 100 is a so-called elastic body in which the compressive elastic modulus of the biocompatible member 100 is not less than 5 kPa and the storage modulus is greater than the loss modulus, the biocompatible member 100 can be said to have properties such that the biocompatible member 100 can maintain its shape even when including the liquid having water as a major component. Also, the biocompatible member 100 can maintain its shape even when lifted by pinching with tweezers, etc. It is therefore easy to handle the biocompatible member 100.

Here, when the fiber 6 is formed of a biologically derived material, and when the biocompatible member 100 that includes the fibers 6 is disposed *in vivo,* the biologically derived material is consumed by the metabolism of the living body, causing the fibers 6 to unravel. In such a case, the bonding force between the adjacent fibers 6 is weak when the adjacent fibers 6 are bonded to each other by interactions other than covalent bonding such as intermolecular forces, hydrophobic interactions, hydrogen bonds, etc. When the bonding force between the adjacent fibers 6 is weak, the biocompatible member 100 is easily consumed by reactions *in vivo.*

In such a case, there are also cases where, according to the application of the biocompatible member 100, it is favorable for the biocompatible member 100 to be consumed promptly; however, there are also cases where it is favorable for the biocompatible member 100 to remain for a relatively long period and maintain the mechanical strength in an *in vivo* environment (e.g., 37 °C).

For example, it is favorable for a cell culture scaffolding or the like to remain for a relatively long period and maintain the mechanical strength in an *in vivo* environment.

Also, when the biocompatible member 100 is disposed *in vivo,* there are cases where pressure and/or heat is applied from the surrounding biological tissue to the biocompatible member 100. In such a case, when the bonding force between the adjacent fibers 6 is weak, gaps tend to occur between the stacked regions. When gaps occur between the stacked regions, the biocompatible member 100 is easily consumed. However, there are cases where it is favorable for the biocompatible member 100 to remain for a relatively long period and maintain the mechanical strength even when the biocompatible member 100 is disposed *in vivo.*

In the biocompatible member 100 according to the embodiment, the adjacent fibers 6 are covalently bonded to each other at multiple locations inside the biocompatible member 100. Therefore, the bonding force between the adjacent fibers 6 can be increased, and so unraveling of the fibers 6 can be suppressed even when the biocompatible member 100 is disposed in an *in vivo* environment. As a result, the longevity of the biocompatible member 100 can be improved and the mechanical strength of the biocompatible member 100 can be maintained in an *in vivo* environment.

For example, as described below, the biocompatible member 100 can maintain its shape even when immersed in a culture medium for 6 days at 37 °C.

Also, the biocompatible member 100 has high compatibility with the cellular tissue at the periphery. Therefore, for example, if the biocompatible member 100 is disposed at a damaged tissue part or in a space after tumor excision, the biocompatible member 100 can be used as scaffolding of cell proliferation, or used to promote healing of a wound and/or tissue reconstruction. Also, there are cases where the biocompatible member 100 is used as a covering member when implanting an artifact and/or drug inside a body. If the biocompatible member 100 is used as a covering member, inflammation inside the body can be suppressed. Also, there are cases where the biocompatible member 100 is used as scaffolding when growing cells/tissue *ex vivo.* If the biocompatible member 100 is used as scaffolding, the cells/tissue can be grown/cultured efficiently or three-dimensionally.

In such applications as well, there are cases where it is favorable for the biocompatible member 100 to remain for a relatively long period and maintain the mechanical strength.

The biocompatible member 100 according to the embodiment can be favorably used in such applications.

### (Method for manufacturing biocompatible member)

A method for manufacturing the biocompatible member 100 according to the embodiment will now be described.

First, an electrospinning apparatus 101 is used to form the fibers 6 and form a deposited body 7 by depositing the fibers 6 that are formed. Also, when depositing the formed fibers 6, the extension directions of the fibers 6 in the deposited body 7 are made uniform as much as possible by mechanically pulling the fibers 6 in one direction. Also, the distance between the fibers 6 is made as small as possible.

FIG. 2 is a schematic view illustrating the electrospinning apparatus 101.

As shown in FIG. 2, the electrospinning apparatus 101 includes a nozzle 102, a power supply 103, and a collection part 104.

The power supply 103 charges a raw material liquid 110 by applying a voltage to the nozzle 102.

The raw material liquid 110 is a liquid in which a biocompatible material is dissolved in a solvent. The biocompatible material is, for example, collagen. The solvent is, for example, water, an alcohol, etc.

The collection part 104 is, for example, cylindrical and rotates in one direction. Also, a voltage of the reverse polarity of the voltage applied to the nozzle 102 may be applied to the collection part 104.

The charged raw material liquid 110 is drawn out toward the collection part 104 by an electrostatic force. The drawn out raw material liquid 110 is elongated, and the solvent included in the raw material liquid 110 is volatilized, thereby forming the fiber 6. At this time, the orientations of the molecules of the biocompatible material included in the raw material liquid 110 are somewhat uniform in the longitudinal direction of the fibers. The formed fibers 6 are deposited on the rotating collection part 104. At this time, the extension directions of the fibers 6 in the deposited body 7 are somewhat uniform because the fibers 6 are mechanically pulled in one direction. Also, the distance between the fibers 6 can be reduced.

A case is illustrated where the fibers 6 are pulled in one direction by the rotating collection part 104; however, for example, the fibers 6 also can be pulled in one direction by a gas flow by causing a gas to flow in the direction in which the fibers 6 are to be drawn out.

Then, deposited bodies 7a to 7c are cut out from the deposited body 7 to include the fibers 6 extending in the desired directions.

FIG. 3 is a schematic view illustrating the cutouts of the deposited bodies 7a to 7c.

As shown in FIG. 3, the deposited bodies 7a to 7c that include the fibers 6 extending in the desired directions can be obtained by cutting out the deposited bodies 7a to 7c while changing the rotation direction of the deposited bodies 7a to 7c as referenced to the extension direction of the fibers 6 in the deposited body 7.

For example, the deposited body 7a includes the fibers 6 extending in the X-direction. The deposited body 7b includes the fibers 6 extending in the Y-direction. The deposited body 7c includes the fibers 6 extending to be tilted 45° with respect to the X-direction.

A case will now be described as an example in which the biocompatible member 100 is manufactured using the deposited bodies 7a and 7b.

FIGS. 4 to 7 are schematic views illustrating processes of manufacturing the biocompatible member 100 using the deposited bodies 7a and 7b.

First, as shown in FIG. 4, the deposited bodies 7a and 7b are alternately stacked on a base member 112.

Although the composition and shape of the base member 112 are not particularly limited, it is favorable for the composition and shape to be such that the adhesion between the stacked deposited bodies 7a and 7b is good. The material of the base member 112 may be, for example, a synthetic polymer such as polystyrene (PS), polyethylene (PE), polypropylene (PP), polymethyl methacrylate (PMMA), polylactic acid (PLA), polyethylene oxide (PEO), polyvinyl alcohol (PVA), polyacrylonitrile (PAN), polyethylene terephthalate (PET), polycarbonate (PC), polyurethane (PU), silicone, or the like, a natural polymer such as collagen, gelatin, laminin, chitosan, chitin, or the like, a metal such as stainless steel, titanium, aluminum, or the like, a ceramic such as silicon dioxide, alumina, titania, zirconia, or the like, a biological tissue such as decellularized tissue, human skin, a tooth, an organ, etc. Also, a biocompatible member 100 that is already manufactured may be used as the base member 112. A member such as a substrate, cloth, sphere, rod, tube, etc., formed by molding the biocompatible member 100 may be used as the base member 112. An artificial biological structure such as an artificial blood vessel, an artificial valve, an artificial joint, an artificial tooth, an artificial mini-organ such as an organoid or the like, artificial meat such as cultured meat, plant-based meat, etc., may be used as the base member 112. The base member 112 may further include a drug, cells, blood, a body fluid, serum, etc.

Then, as shown in FIG. 5, a volatile liquid is caused to penetrate the stacked deposited bodies 7a and 7b.

Although not particularly limited, it is favorable for the volatile liquid not to dissolve the fiber 6 as much as possible. For example, water, an alcohol (ethanol, methanol, isopropyl alcohol, butanol, or the like), an alcohol aqueous solution, a ketone (acetone, ethyl methyl ketone, or the like), etc., are used.

For example, a waste cloth 113 including ethanol is placed on the deposited bodies 7a and 7b placed on the base member 112. Then, the ethanol that is included in the waste cloth 113 penetrates the deposited bodies 7a and 7b. In such a case, the penetration amount of the ethanol can be adjusted to form micro air bubbles (spaces not filled with the volatile liquid) inside the deposited bodies 7a and 7b. The waste cloth 113 may be detached when a prescribed amount of ethanol has penetrated the deposited bodies 7a and 7b.

Furthermore, as shown in FIG. 6, an aggregate 17 also can be formed by placing a base member 112b, which is similar to the base member 112, and a weight 114 on the deposited bodies 7a and 7b, and by volatilizing the penetrated volatile liquid while compressing the deposited bodies 7a and 7b in the stacking direction. As a result, the aggregate 17 such as that shown in FIG. 7 can be formed in which the adhesion in the stacking direction is increased while suppressing contraction in the planar direction. Volatilization and drying may be performed without placing the base member 112b and/or the weight 114 on the deposited bodies 7a and 7b.

As described above, the extension directions of the fibers 6 included in the deposited bodies 7a and 7b are somewhat uniform, and the distance between the fibers 6 is somewhat small. Therefore, the capillary force of the liquid more easily acts between the adjacent fibers 6 when volatilizing the volatile liquid. The adjacent fibers 6 are closely adhered to each other by the action of the capillary force of the liquid. In such a case, for example, the fibers 6 are partially bonded to each other by interactions other than covalent bonding. The interactions other than covalent bonding are, for example, intermolecular forces, hydrophobic interactions, hydrogen bonds, etc.

The fibers 6 aggregate three-dimensionally in the aggregate 17. Also, if the micro air bubbles described above are formed in the deposited bodies 7a and 7b, voids can form in the aggregate 17 after volatilizing the volatile liquid. The thickness of the aggregate 17 closely adhered to the base member 112 is about 2/3 to 1/5 of the thickness of the deposited bodies 7a and 7b before being impregnated with the volatile liquid. Also, the aggregate 17 becomes transparent or semi-transparent.

Here, in the aggregate 17, the adjacent fibers 6 are partially bonded to each other by interactions other than covalent bonding. Therefore, as described above, it is difficult to increase the bonding force between the adjacent fibers 6. If the bonding force between the adjacent fibers 6 cannot be increased, the aggregate 17 will be consumed in a relatively short period and/or the mechanical strength of the aggregate 17 will decrease in a relatively short period in an *in vivo* environment.

Therefore, next, the adjacent fibers 6 are covalently bonded to each other to improve the longevity and maintain the mechanical strength in the *in vivo* environment. The covalent bonds between the adjacent fibers 6 can be made by thermal crosslinking. When, however, simply thermal crosslinking is performed, there is a risk that the surface of the fiber 6 may be modified (e.g., oxidized), and the biocompatibility may degrade.

Therefore, according to the method for manufacturing the biocompatible member 100 according to the embodiment, thermal crosslinking treatment (so-called vacuum thermal crosslinking treatment) of the aggregate 17 is performed in an environment decompressed from atmospheric pressure.

For example, the thermal crosslinking treatment in the environment decompressed from atmospheric pressure can be performed by exposing the aggregate 17 to an atmosphere having a pressure of not more than 10 Pa and a temperature of about 100 °C to 200 °C for about 10 minutes to 48 hours.

In such a case, it is favorable to dry the aggregate 17 before the thermal crosslinking treatment because the thermal crosslinking in the environment decompressed from atmospheric pressure is dehydration-condensation reactions by amino groups included in collagen, etc. Because the liquid used when forming the aggregate 17 is a volatile liquid, for example, the aggregate 17 can be dried by natural drying, etc. However, there is a risk that the surface of the fibers 6 may be modified if the aggregate 17 is left for a long period of time in ambient air.

Therefore, if necessary, dry air drying, vacuum drying, freeze-drying treatment, etc., of the aggregate 17 can be performed before the thermal crosslinking treatment. For example, the aggregate 17 may be frozen by immersion in liquid nitrogen, etc., followed by sublimating the ice in a high vacuum.

Here, thermal crosslinking occurs more easily as the distance between the portions of the fibers 6 to be crosslinked decreases. Therefore, for example, even when directly performing thermal crosslinking treatment of the deposited bodies 7a to 7c made by mechanically pulling the fibers 6, it is difficult to increase the number of locations at which the adjacent fibers 6 are covalently bonded to each other (see FIG. 10).

As described above, in the aggregate 17, the adjacent fibers 6 are substantially closely adhered to each other by the capillary force of the liquid. Also, the adjacent fibers 6 are partially bonded to each other by interactions other than covalent bonding. Therefore, if thermal crosslinking treatment of the aggregate 17 is performed in an environment decompressed from atmospheric pressure, the adjacent fibers 6 can be covalently bonded to each other at more locations in heat treatment having a low temperature or short period of time. The bonding force increases as the number of covalently-bonded locations increases, and therefore, as described above, the longevity of the biocompatible member 100 can be improved and the mechanical strength of the biocompatible member 100 can be maintained in an *in vivo* environment.

Generally, the fibers 6 include adsorbed water and/or bound water. Also, as described above, the aggregate 17 includes micro voids inside the aggregate 17. Here, when the aggregate 17 is heated in a decompressed environment, moisture desorbed by a thermal dehydration reaction or a gas (e.g., air) included inside the voids undergoes thermal expansion. Therefore, for example, the closely-adhered state of the adjacent fibers 6 and the deposited bodies 7a and 7b can be adjusted so that the gas that is produced escapes outside from the interior of the aggregate 17. Also, hydrostatic pressure treatment and freeze-drying treatment of the aggregate 17 can be performed before the thermal crosslinking treatment.

An aggregate 17a that is subjected to thermal crosslinking treatment can be detached from the base member 112 and used as the biocompatible member 100, or can be used as the biocompatible member 100 as-is as one piece with the base member 112.

In other words, the biocompatible member 100 (the aggregate 17a that is subjected to thermal crosslinking treatment) can be manufactured as described above.

Thus, the manufactured biocompatible member 100 is in a dry state. It is therefore easy to store and/or transport the biocompatible member 100.

On the other hand, as described above, the biocompatible member 100 includes a liquid having water as a major component when the biocompatible member 100 is used.

Therefore, hydration treatment of the aggregate 17a that is subjected to thermal crosslinking treatment also can be performed as necessary.

For example, the hydration treatment can be performed by immersing the aggregate 17a in a liquid having water as a major component and leaving to stand, supplying a liquid having water as a major component to the aggregate 17a, or making a mist of a liquid having water as a major component and spraying the mist onto the aggregate 17a. The liquid having water as a major component penetrates the voids and/or gaps between the fibers 6; and the fibers 6 absorb the water and swell. When the hydration treatment is performed, the aggregate 17a swells twofold to five-fold in a room-temperature environment. To promote the penetration of the liquid into voids and/or gaps, decompression treatment, hydrostatic pressure treatment, ultrasonic treatment, etc., can be performed.

Thus, the biocompatible member 100 that includes a liquid having water as a major component can be manufactured.

Here, there are cases where it is favorable for the aggregate 17a to swell sufficiently when performing hydration treatment. In such a case, hydrostatic pressure treatment can be performed on the aggregate 17a that is subjected to thermal crosslinking treatment.

FIG. 8 is a schematic view illustrating hydrostatic pressure treatment.

As shown in FIG. 8, the aggregate 17a that is subjected to thermal crosslinking treatment and a liquid 116 having water as a major component are sealed in an airtight bag 117. The airtight bag 117 in which the aggregate 17a and the liquid 116 are sealed is placed inside a chamber 119 to which a pump 118 is connected.

Then, the pump 118 supplies air 120 to the interior of the chamber 119 to increase the internal pressure of the chamber 119. As the internal pressure of the chamber 119 increases, the liquid 116 and the aggregate 17a are pressurized via the airtight bag 117. A piston can be used instead of the pump 118. Also, a liquid pressure medium such as water, propylene glycol, etc., can be used instead of the air 120. For example, the internal pressure of the chamber 119 can be set to be not less than 5 MPa and not more than 1 GPa, and favorably not less than 100 MPa and not more than 1 GPa. By pressurizing the liquid 116, the liquid 116 isotropically penetrates into the voids and/or gaps between the fibers 6. Also, the air that is present between the fibers 6 is discharged from the aggregate 17a. Thus, hydrostatic pressure treatment (hydration treatment) can be performed on the aggregate 17a that is subjected to thermal crosslinking treatment.

Swelling of the aggregate 17a can be promoted by performing hydrostatic pressure treatment. Also, by performing hydrostatic pressure treatment, compared to the hydration treatment described above, hydration of the fibers 6 can be promoted, and the aggregate 17a can efficiently include the liquid 116. Also, by performing the hydrostatic pressure treatment, bacteria included in the biocompatible member 100 (the aggregate 17a) is reduced. By sterilizing the biocompatible member 100, the biocompatibility of the biocompatible member 100 is improved.

Also, decompression treatment can be performed on the aggregate 17a that is subjected to thermal crosslinking treatment.

FIG. 9 is a schematic view illustrating decompression treatment.

As shown in FIG. 9, the aggregate 17a that is subjected to thermal crosslinking treatment and the liquid 116 having water as a major component are stored inside a chamber 121. Then, a vacuum pump or the like is used to decompress the interior of the chamber 121. For example, the internal pressure of the chamber 121 is set to be less than 1 atmosphere. By decompressing the liquid 116, the air that is present between the fibers 6 is discharged from the aggregate 17a, and the liquid 116 penetrates between the fibers 6. Thus, decompression treatment (hydration treatment) can be performed on the aggregate 17a that is subjected to thermal crosslinking treatment.

The hydrostatic pressure treatment described above may be performed after the decompression treatment is performed.

As described above, the method for manufacturing the biocompatible member 100 according to the embodiment can include the following processes:
A process of causing a volatile liquid to penetrate a deposited body.

A process of volatilizing the liquid that penetrated the deposited body.

A process of performing, in an environment decompressed from atmospheric pressure, thermal crosslinking treatment on the deposited body after the liquid is volatilized.

In such a case, the process of forming the deposited body includes pulling the fibers in one direction and making the extension direction of the deposited fibers uniform.

In the process of causing the volatile liquid to penetrate, the distance between adjacent fibers is reduced by the capillary force acting between the adjacent fibers.

The process of performing the thermal crosslinking treatment includes covalently bonding the fibers having the reduced distance to each other at multiple locations.

Also, in the process of causing the volatile liquid to penetrate, spaces that are not filled with the volatile liquid can be formed in the deposited body.

Also, a process of performing hydrostatic pressure treatment and a process of performing freeze-drying treatment can be further included before performing thermal crosslinking treatment.

Also, a process of performing hydrostatic pressure treatment on the deposited body that is subjected to thermal crosslinking treatment can be further included.

### (Test example)

Test examples will now be described.

In the test examples, collagen, which is a biologically derived material, was used as a biocompatible material. Ethanol was used as a volatile liquid.

For the test examples, the biocompatible member 100 was manufactured using the method for manufacturing the biocompatible member 100 described above.

### (Appearance observation)

Samples at each process of the manufacture of the biocompatible member 100 were observed by SEM (Scanning Electron Microscope: scanning electron microscope).

FIG. 10 is a SEM photograph of the surface of the deposited body 7a. The deposited bodies 7b and 7c were formed to be similar.

As described above, the deposited body 7a was cut out from the deposited body 7 formed by electrospinning. When forming the deposited body 7 by electrospinning, the fibers 6 were mechanically pulled in one direction so that the extension directions of the fibers 6 were as uniform as possible. Also, the distance between the fibers 6 was set to be as small as possible.

However, even when the fibers 6 are mechanically pulled in one direction, there is a limit on making the extension directions of the fibers 6 uniform and reducing the distance between the fibers 6. Therefore, it can be seen from FIG. 10 that it is difficult to closely adhere the multiple fibers 6.

As described above, thermal crosslinking occurs easily as the distance between the portions of the fibers 6 to be crosslinked decreases. Therefore, even if thermal crosslinking treatment is performed on the fibers 6 in a state such as that shown in FIG. 10, it is difficult to increase the number of locations at which the adjacent fibers 6 are covalently bonded to each other.

That is, even when thermal crosslinking treatment is performed on a deposited body formed by electrospinning, it is difficult to improve longevity and maintain mechanical strength in an *in vivo* environment.

FIGS. 11A and 11B are SEM photographs of the surface of the deposited body 7a after the treatment with ethanol. FIG. 11A is when treatment is performed to provide strong adhesion between the deposited body 7a and the base member 112b; and FIG. 11B is when treatment is performed to provide weak adhesion between the deposited body 7a and the base member 112b.

The deposited bodies 7b and 7c were formed to be similar.

As described with reference to FIG. 10, the extension directions of the fibers 6 included in the deposited body 7a were somewhat uniform, and the distance between the fibers 6 also was somewhat small. Therefore, when ethanol was volatilized, it was easier for the capillary force of the ethanol to act between the adjacent fibers 6.

Therefore, as shown in FIGS. 11A and 11B, the adjacent fibers 6 were closely adhered to each other by the capillary force of the ethanol. Also, as described above, the fibers 6 were partially bonded to each other by interactions other than covalent bonding. The interactions other than covalent bonding were, for example, intermolecular forces, hydrophobic interactions, hydrogen bonds, etc.

### (Polarized FT-IR-ATR method)

In an elongated polymer material, the extension direction of the major axes of the molecules (the molecular axes) tends to be the extension direction of the polymer material (the fibers). Therefore, by determining the extension direction of the major axes of the molecules at the surface of the biocompatible member 100, the extension directions of the fibers 6 can be estimated, and in turn, it can be determined whether or not the fibers 6 are oriented. The fibers 6 extending in about the same direction is referred to as the fibers 6 being "oriented".

The extension direction of the major axes of the molecules can be determined by using a structure determination technique corresponding to the type of polymer material. For example, Raman spectroscopy can be used in the case of polystyrene, etc., and polarized absorption spectroscopy can be used in the case of polyimide, etc. As an example in the test examples, a case will be described where the polymer material is an organic compound with an amide group, such as collagen, etc. When the organic compound includes an amide group, for example, the extension direction of the major axes of the molecules can be determined by using the polarized FT-IR-ATR method (Fourier Transform-Infrared Spectroscopy- Attenuated Total Reflectance: Fourier transform-infrared spectroscopy-attenuated total reflectance method), which is one type of infrared spectroscopy.

An analysis method using the polarized FT-IR-ATR method will now be described.

The absorption intensity of light of a wave number of 1,640 cm⁻¹ is taken as *T1*; and the absorption intensity of light of a wave number of 1,540 cm⁻¹ is taken as T2. The absorption intensity T1 is the absorption intensity in a direction orthogonal to the extension direction of the major axes of the molecules. The absorption intensity T2 is the absorption intensity in the extension direction of the major axes of the molecules. Therefore, if the absorbance ratio (*T1*/*T2*) in the prescribed polarization direction becomes small, it can be determined that many molecules extend in the polarization direction.

Then, the absorbance ratio (*T1*/*T2*) was measured while modifying the angle with the polarization direction for samples in which the biocompatible member 100 after the hydration treatment was freeze-dried. An absorbance ratio at which the value was a maximum was taken as R1; and the absorbance ratio at which the value was minimum was taken as R2. For example, the orientation of the biocompatible member 100 at which the absorbance ratio R2 was obtained was rotated 90° with respect to the orientation of the biocompatible member 100 at which the absorbance ratio R1 was obtained. Then, the ratio (R1/R2) was used as an orientation degree parameter. The degree of the orientation increases as the orientation degree parameter (*R1*/*R2*) increases.

In a sample in which the biocompatible member 100 after the hydration treatment was freeze-dried, the value of the orientation degree parameter (R1/R2) was about 1.10. Thus, it was confirmed that the fibers 6 were oriented in the biocompatible member 100 after the hydration treatment.

FIGS. 12A and 12B are SEM photographs of the surface of the aggregate 17 on which hydrostatic pressure treatment and freeze-drying treatment were performed. It can be seen from FIGS. 12A and 12B that voids having diameters of not less than 10 µm were formed at the surface of the biocompatible member 100 after the hydration treatment at a density of not less than 1/mm².

### (Compressive elastic modulus)

A constant speed compression test using a creep meter was performed on the biocompatible member 100. The thickness of the biocompatible member 100 was set to 1 to 4 mm; the temperature of the vacuum thermal crosslinking treatment was set to 140 °C; and the time was set to the two levels of 1 h and 4 h. Vacuum thermal crosslinking treatment was not performed for the comparative example 1. Three samples formed by stamping 8 mm diameter test pieces were made and measured for each level. For all of the measurements, the compression rate was set to 0.05 mm/s; and the maximum strain applied was set to 50%. The measurement temperature was set to 20 °C.

FIG. 13 is a table showing measurement results of the compressive elastic modulus of the biocompatible member 100 according to the test examples.

As shown in FIG. 13, in the measurement results of three samples (examples 1 to 3), a compressive elastic modulus E0 for a strain of 0 to 10% was 0.35 to 0.55 kPa. The compressive elastic modulus Em for a strain of 42 to 48% was 8.5 to 11.3 kPa.

### (Dynamic viscoelasticity measurement)

The viscoelasticity of the biocompatible member 100 was measured using a dynamic viscoelastic method of the compression mode in water. The thickness of the biocompatible member 100 was set to 3 mm; the temperature increase range was set from room temperature (e.g., 24 °C) to 90 °C; the temperature raising rate was set to 2 °C/min; and the viscoelasticity was measured while raising the temperature. The two levels of distilled water or physiologic saline solution were used for the immersion liquid. Distilled water for high performance liquid chromatograph made by FUJIFILM Wako Chemicals was used as the distilled water; and physiological saline solution PL "Fuso" made by Fuso Pharmaceutical Industries was used as the physiologic saline solution. A sinusoidal force was applied to the biocompatible member 100; the storage modulus E' and the loss modulus E" for a strain of 0.17 to 7% were measured; and the complex elastic modulus E* was calculated. The frequency of the sinusoidal force was set to 1 Hz.

FIG. 14A is a graph showing measurement results performed in distilled water. The horizontal axis is the temperature; the vertical axis is the storage modulus and the loss modulus; and the storage modulus and loss modulus-temperature curves of the biocompatible member 100 according to the test examples are shown. FIG. 14B is a graph showing measurement results performed in a physiologic saline solution. This graph shows the storage modulus and loss modulus-temperature curves of the biocompatible member 100 according to the test examples, in which the horizontal axis is the frequency, and the vertical axis is the storage modulus and the loss modulus.

As shown in FIGS. 14A and 14B, the decrease of the elastic modulus for the biocompatible member 100 was less than that of the comparative examples for which vacuum thermal crosslinking treatment was not performed over the entire temperature range of 37 °C and higher. At this time, the fluctuation of the chuck-to-chuck distance of the device corresponding to the sample thickness when measuring was stable within ±10% in the range from the start to the end of the measurement. For the comparative examples 1 to 4, the chuck-to-chuck distance decreased in the region of 40 °C and higher; and the chuck-to-chuck distance at the end of the measurement was about 43 to 46% of the initial. It was inferred that softening due to heating occurred, and data of 40 °C and higher is not shown.

FIG. 15 is a table summarizing the results of the dynamic viscoelasticity measurement in water of FIGS. 14A and 14B at a temperature of 37 °C. At 37 °C, the storage modulus was greater than the loss modulus. Therefore, the biocompatible member 100 maintains its properties as an elastic body. The thickness retention rate of the sample when measuring, estimated from the chuck-to-chuck distance when measuring, was 95 to 98%.

### (Immersion test)

The aggregate 17, which was treated with ethanol but not subjected to thermal crosslinking treatment, and the aggregate 17a (the biocompatible member 100), which was treated with ethanol and subjected to thermal crosslinking treatment, were subjected to hydration treatment with Milli-Q water, and then stamped into 8 mm diameter test pieces. The test pieces were immersed in a culture medium and stored at a temperature of 37 °C; and the progress was observed. 700 µL each of DMEM (1% PS and 10 FBS) was used as the culture medium. The longevity was evaluated for the aggregates 17 and 17a by using the naked eye, a phase contrast microscope, and a stereo microscope. Also, the water content by weight was determined by using an electronic force balance to measure the weight of the aggregate 17a after thermal crosslinking treatment, after hydration, and after the immersion test.

FIG. 16 is a table showing results of the immersion test of the aggregates 17 and 17a. The test pieces that were confirmed to maintain their shape and remain even after immersion for 6 days are marked with circles; the test piece confirmed to remain but to be contracted after the immersion are marked with triangles; and the test pieces confirmed to be difficult to observe and to be consumed after the immersion are marked with ×.

The aggregate 17 that was not subjected to thermal crosslinking treatment was consumed in about 1 day.

The aggregate 17a that was subjected to thermal crosslinking treatment maintained the form of the test piece even after 6 days.

The results show that compared to the aggregate 17 that was not subjected to thermal crosslinking treatment, the aggregate 17a that was subjected to thermal crosslinking treatment had a greater bonding force between the adjacent fibers 6 and did not decompose easily. Also, it was estimated that the biocompatible member 100 could remain to some extent and maintain mechanical strength *in vivo* even when stress and/or heat was applied to the biocompatible member 100 *in vivo.*

### (Subcutaneous transplant in rats)

The biocompatible member 100 (the aggregate 17a that was subjected to thermal crosslinking treatment) was subcutaneously transplanted into rats; and the state was observed by naked eye after 1 day, after 4 days, and after 7 days. The quantity N was set to 3.

As a result, in the aggregate 17a that was subjected to vacuum thermal crosslinking treatment for 4 h, the biocompatible member 100 remained and did not become a gel even 4 days after transplant. Even 6 days after transplant, one out of three remained and maintained its shape.

In the aggregate 17a that was subjected to vacuum thermal crosslinking treatment for 1 h, the biocompatible member 100 remained and did not become a gel even 1 day after transplant. The aggregate 17a was consumed at the timing of 4 days after transplant.

No rejection reaction such as inflammation or the like was observed during this period.

According to embodiments above, a biocompatible member and a method for manufacturing a biocompatible member can be realized in which longevity can be improved and mechanical strength can be maintained in an *in vivo* environment.

While certain embodiments have been described, these embodiments have been presented by way of example only, and are not intended to limit the scope of the inventions. Indeed, the novel embodiments described herein may be embodied in a variety of other forms; furthermore, various omissions, substitutions, and changes in the form of the embodiments herein may be made without departing from the spirit of the inventions. The accompanying claims and their equivalents are intended to cover such forms or modifications as would fall within the scope and spirit of the inventions. Moreover, above-mentioned embodiments can be combined mutually and can be carried out.

### [Reference Numeral List]

6 fiber
7 deposited body
7a to 7c deposited body
17 aggregate
17a aggregate
100 biocompatible member

## Claims

1. A biocompatible member (100), comprising:
a plurality of fibers (6) including a biocompatible material,
mutually-adjacent fibers (6) among the fibers (6) being covalently bonded at a plurality of locations in an extension direction of the fibers (6).

2. The biocompatible member (100) according to claim 1, wherein
the fibers (6) extend in a first direction in a first region,
the fibers (6) extend in a second direction crossing the first direction in a second region,
the first region and the second region are stacked, and at a boundary between the first region and the second region, a plurality of the fibers (6) adjacent to one of the fibers (6) in a stacking direction is covalently bonded to the one of the fibers (6).

3. The biocompatible member (100) according to claim 1 or 2, wherein
the covalent bonding is performed by crosslinking,
the biocompatible member (100) does not include a chemically crosslinked component, and
adjacent fibers (6) among the fibers (6) in the biocompatible member (100) are thermally crosslinked.

4. The biocompatible member (100) according to any one of claims 1 to 3, wherein
a shape of the biocompatible member (100) is maintained even when immersed in a culture medium for 6 days at 37 °C.

5. The biocompatible member (100) according to any one of claims 1 to 4, wherein, further comprising:
a liquid having water as a major component,
a content ratio of the liquid being not less than 40 mass% and not more than 90 mass%.

6. The biocompatible member (100) according any one of claims 1 to 5, wherein
voids having diameters of not less than 10 µm are provided in a surface of the biocompatible member (100) at a density of not less than 1/mm².

7. The biocompatible member (100) according to any one of claims 1 to 6, wherein
the biocompatible material is collagen.

8. A method for manufacturing a biocompatible member (100), comprising:
forming a deposited body (7) by depositing fibers (6) including a biocompatible material;
causing a liquid to penetrate the deposited body (7), the liquid being volatile;
volatilizing the liquid that penetrated the deposited body (7); and
performing thermal crosslinking treatment of the deposited body (17) after the liquid is volatilized in an environment decompressed from atmospheric pressure,
the forming of the deposited body (7) including making extension directions of the deposited fibers (6) uniform by pulling the fibers (6) in one direction,
the penetration of the volatile liquid including reducing a distance between mutually-adjacent fibers (6) among the fibers (6) by a capillary force acting between the mutually-adjacent fibers (6) among the fibers (6),
the thermal crosslinking treatment including covalently bonding the fibers (6) having the reduced distance to each other at a plurality of locations.

9. The method for manufacturing the biocompatible member (100) according to claim 8, wherein
the penetration of the volatile liquid includes forming a space in the deposited body (7) not filled with the volatile liquid.

10. The method for manufacturing the biocompatible member (100) according to claim 8 or 9, further comprising:
performing hydrostatic pressure treatment of the deposited body (17) after the thermal crosslinking treatment.
